(19) Europäisches Patentamt
European Patent Office
Office européen des brevets

(11) **EP 3 165 564 B1**

(12) **EUROPEAN PATENT SPECIFICATION**

(45) Date of publication and mention
of the grant of the patent:
**20.11.2019 Bulletin 2019/47**

(51) Int Cl.:
*A01N 25/10* (2006.01)     *A01N 59/20* (2006.01)
*B01J 20/26* (2006.01)     *B01J 20/30* (2006.01)
*C08J 3/24* (2006.01)     *C08K 3/00* (2018.01)
*C08K 3/22* (2006.01)

(21) Application number: **16827904.0**

(22) Date of filing: **22.03.2016**

(86) International application number:
**PCT/KR2016/002851**

(87) International publication number:
**WO 2017/014400 (26.01.2017 Gazette 2017/04)**

(54) **SUPERABSORBENT POLYMER HAVING EXCELLENT ANTIMICROBIAL AND DEODORIZING PROPERTIES, AND METHOD FOR PREPARING SAME**

SUPERSAUGFÄHIGES POLYMER MIT HERVORRAGENDER ANTIMIKROBIELLEN UND DESODORIERENDEN EIGENSCHAFTEN SOWIE VERFAHREN ZUR HERSTELLUNG DAVON

POLYMÈRE SUPERABSORBANT PRÉSENTANT DES PROPRIÉTÉS DÉSODORISANTES ET ANTIMICROBIENNES, ET SON PROCÉDÉ DE PRÉPARATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.07.2015 KR 20150101781**

(43) Date of publication of application:
**10.05.2017 Bulletin 2017/19**

(73) Proprietors:
• **LG Chem, Ltd.**
  **Seoul 07336 (KR)**
• **Seoul National University R&DB Foundation**
  **Gwanak-gu**
  **Seoul**
  **08826 (KR)**

(72) Inventors:
• **JANG, Min-Seok**
  **Daejeon 34122 (KR)**
• **KIM, Young Sam**
  **Daejeon 34122 (KR)**
• **CHOI, Yong Seok**
  **Daejeon 34122 (KR)**
• **LEE, Jong-Chan**
  **Seoul 06522 (KR)**
• **HONG, Hyunkee**
  **Seoul 05114 (KR)**
• **KIM, Na Kyung**
  **Seoul 08210 (KR)**

(74) Representative: **Goddar, Heinz J.**
**Boehmert & Boehmert**
**Anwaltspartnerschaft mbB**
**Pettenkoferstrasse 22**
**80336 München (DE)**

(56) References cited:
EP-A1- 1 616 911     JP-A- H09 143 850
KR-A- 20060 002 960     KR-A- 20140 145 810
KR-A- 20150 064 712     US-A1- 2007 048 344
US-A1- 2014 221 948     US-A1- 2014 221 948
US-A1- 2015 181 863

• K Gopalakrishnan ET AL: "ANTIBACTERIAL ACTIVITY OF Cu 2 O NANOPARTICLES ON E.COLI SYNTHESIZED FROM TRIDAX PROCUMBENS LEAF EXTRACT AND SURFACE COATING WITH POLYANILINE", Digest Journal of Nanomaterials and Biostructures, 1 April 2012 (2012-04-01), pages 833-839, XP055528371, Retrieved from the Internet: URL:http://chalcogen.ro/833_Gopalakrishnan .pdf [retrieved on 2018-11-29]

**Description**

**Technical Field**

The present invention relates to a method of preparing a superabsorbent resin having excellent antibacterial and deodorizing properties.

**Background Art**

Superabsorbent resins (or superabsorbent polymers, SAPs) are synthetic polymer materials that are able to absorb about 500 to 1000 times their own weight in moisture, and have been widely applied to hygiene products, such as disposable baby and adult diapers and the like. Such superabsorbent resins in the diapers function to absorb and retain urine.

A superabsorbent resin may be prepared using a reversed-phase suspension polymerization process or an aqueous solution polymerization process. A hydrogel polymer obtained through a polymerization process is typically sold in the form of a powder product resulting from drying and pulverizing processes. As such, a fine powder having a particle size of about 150 $\mu$m or less, falling out of the normal particle size range, may be generated during the pulverizing of the dried polymer. The fine powder is recycled in the form of a mixture with a hydrogel polymer in current processing because it cannot be sold as a normal product. However, the addition of fine-powder granulates results in deteriorated properties of the superabsorbent resin and lowered production efficiency. Hence, there is a need for techniques for efficiently recycling fine-powder granulates.

Moreover, in the case where users wear hygiene products, the matter, such as urine or sweat, may be attached to the surface of a superabsorbent resin, offensive odors may be generated, and exposure to microorganisms such as bacteria and the like is inevitable. As the size of the sanitary market including adult diapers increases, antibacterial and deodorizing effects of diapers are regarded as increasingly important, and the development of superabsorbent resins having such effects is urgent.

Therefore, there has been required a superabsorbent resin having excellent and safe antibacterial and deodorizing properties, which directly interacts with microorganisms to thus exhibit antibacterial and deodorizing effects and in which an antibacterial material itself has no toxicity.

EP 1 616 911 A1 discloses a resin composition comprising a water-absorbing agent and an antibacterial metal. This metal compound is included in an amount of 0.001-1 pbw. Copper is disclosed as antibacterial agent.

JP H09 143850 A discloses a high-water-absorbent antimicrobial sheet comprising a superabsorbent polymer and copper-based antibacterial agents.

US 2014/221948 A1 discloses a hydroactive polymer that has copper or copper(I) or (II) ions admixed.

**Disclosure**

**Technical Problem**

Accordingly, the present invention has been made keeping in mind the above problems encountered in the related art, and the present invention is intended to provide a method of preparing an antibacterial superabsorbent resin, wherein a superabsorbent resin, the properties of which are not deteriorated, is prepared by effectively recycling a fine powder and is imparted with excellent antibacterial and deodorizing effects.

**Technical Solution**

The present invention provides a method of preparing an antibacterial superabsorbent resin.

The present invention provides a method of preparing an antibacterial superabsorbent resin, comprising: 1) drying and pulverizing a hydrogel polymer, and then classifying into a fine powder having a particle size of less than 150 $\mu$m and a base polymer having a particle size of 150 to 850 $\mu$m; 2) mixing the fine powder, water and a copper-based antibacterial agent, thus preparing a fine-powder regranulate; 3) passing the fine-powder regranulate through a chopper and then performing drying and pulverizing; and 4) classifying the pulverized fine-powder regranulate into a superabsorbent resin having a particle size of 150 to 850 $\mu$m, thus obtaining the superabsorbent resin.

Further embodiments are disclosed in the dependent claims.

**Advantageous Effects**

According to the present invention, a fine powder can be recycled in a manner in which the fine powder is added with an additive and an antibacterial material upon regranulation thereof, thus minimizing deterioration of the properties of the resulting superabsorbent resin and imparting antibacterial properties, as a consequence of which an antibacterial superabsorbent resin having antibacterial and deodorizing effects can be economically obtained.

**Description of Drawing**

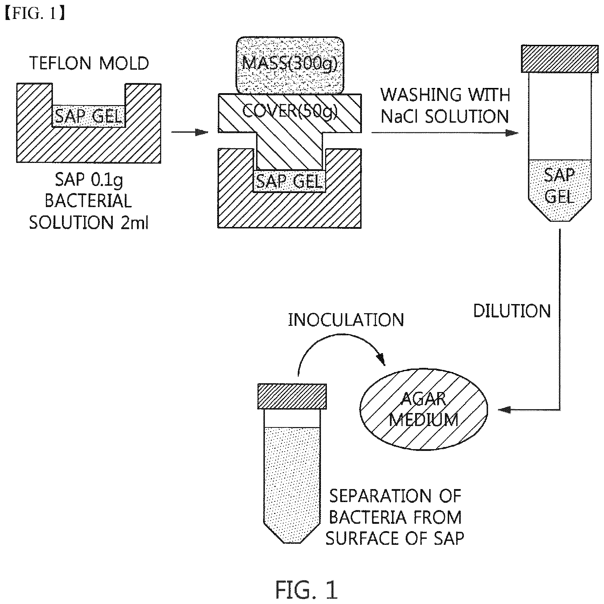

FIG. 1 schematically shows a test process in Test Example 1 according to the present invention.

**Best Mode**

With regard to recycling of a fine powder in the preparation of a superabsorbent resin according to the present invention, a fine-powder regranulate having improved properties is imparted with antibacterial and deodorizing effects, unlike a conventional regranulation process.

In a conventional fine-powder regranulation process, a superabsorbent resin has been prepared in a manner in which an agglomerate, obtained simply by agglomerating a mixture of water and fine powder, is added to a hydrogel polymer. The properties of the superabsorbent resin thus obtained may deteriorate compared to a superabsorbent resin prepared without the use of a fine powder. Furthermore, as the adult diaper market grows, the importance of antibacterial and deodorizing effects of diapers is increasing.

Accordingly, in the present invention, a superabsorbent resin is prepared by efficiently recycling a fine-powder regranulate, and is imparted with antibacterial effects, resulting in a superabsorbent resin having antibacterial and deodorizing properties.

Specifically, provided is an antibacterial superabsorbent resin, comprising a superabsorbent resin and a copper-based antibacterial agent added thereto.

In an embodiment of the present invention, the copper-based antibacterial agent preferably includes $Cu_2O$ (cuprous oxide).

In another embodiment of the present invention, the copper-based antibacterial agent is preferably used in an amount of 0.2 to 0.9 parts by weight, and more preferably 0.5 to 0.8 parts by weight, based on 100 parts by weight of the superabsorbent resin. When the copper-based antibacterial agent is added in a higher concentration, antibacterial effects are increased but absorption under pressure (AUP) and permeability may decrease, ultimately deteriorating the properties of the antibacterial superabsorbent resin. Hence, the amount of the copper-based antibacterial agent that is added is preferably set to fall within the above range.

In still another embodiment of the present invention, the copper-based antibacterial agent exhibits antibacterial activity against gram-negative bacteria, and the gram-negative bacteria may be, but is not limited to, *Escherichia coli.*

In another embodiment of the present invention, the mixing in 2) above may further comprise mixing at least one additive selected from the group consisting of sodium hydroxide (NaOH) and sodium persulfate (SPS).

By virtue of the mixing of the additive, the properties of the superabsorbent resin obtained through recycling of the fine powder may be improved.

In order to prepare the antibacterial superabsorbent resin according to the present invention, a hydrogel polymer may be synthesized through steps and methods typically useful in the art. Specifically, in the preparation of the antibacterial superabsorbent resin according to the present invention, the hydrogel polymer may be obtained by polymerizing a monomer composition comprising a water-soluble ethylenic unsaturated monomer and a polymerization initiator.

For the polymerization initiator included in the monomer composition, depending on the polymerization method, a photopolymerization initiator may be used upon photopolymerization, and a thermal polymerization initiator may be employed upon thermal polymerization. Even when photopolymerization is conducted, a predetermined amount of heat is generated due to irradiation with UV light, and also due to polymerization, which is an exothermic reaction, and thus a thermal polymerization initiator may be additionally included.

In the method of preparing the antibacterial superabsorbent resin according to the present invention, the thermal polymerization initiator is not particularly limited, but preferably includes at least one selected from the group consisting of a persulfate-based initiator, an azo-based initiator, hydrogen peroxide, and ascorbic acid. In particular, examples of the persulfate-based initiator may include sodium persulfate ($Na_2S_2O_8$), potassium persulfate ($K_2S_2O_8$), and ammonium persulfate (($NH_4$)$_2S_2O_8$), and examples of the azo-based initiator may include 2,2-azobis(2-amidinopropane)dihydrochloride, 2,2-azobis-(N,N-dimethylene)isobutyramidine dihydrochloride, 2-(carbamoylazo)isobutyronitrile, 2,2-azobis[2-(2-imidazolin-2-yl)propane]dihydrochloride, and 4,4-azobis-(4-cyanovaleric acid).

In the method of preparing the antibacterial superabsorbent resin according to the present invention, the photopolymerization initiator is not particularly limited, but preferably includes at least one selected from the group consisting of benzoin ether, dialkyl acetophenone, hydroxyl alkylketone, phenyl glyoxylate, benzyl dimethyl ketal, acyl phosphine, and $\alpha$-aminoketone. A specific example of the acyl phosphine may include commercially available Lucirin TPO, that is, 2,4,6-trimethyl-benzoyl-trimethyl phosphine oxide.

In the method of preparing the antibacterial superabsorbent resin according to the present invention, the water-soluble ethylenic unsaturated monomer may be used without particular limitation, so long as it is a monomer typically used to synthesize a superabsorbent resin, and preferably includes at least one selected from the group consisting of an anionic monomer and salts thereof, a nonionic hydrophilic monomer, and an amino group-containing unsaturated monomer and quaternary salts thereof. Specifically useful is at least one selected from the group consisting of anionic monomers and salts thereof, such as acrylic acid, methacrylic acid, maleic anhydride, fumaric acid, crotonic acid, itaconic acid, 2-acryloylethanesulfonic acid, 2-methacryloylethanesulfonic acid, 2-(meth)acryloylpropanesulfonic acid, and 2-(meth)acrylamide-2-methylpropane sulfonic acid; nonionic hydrophilic monomers such as (meth)acrylamide, N-substituted (meth)acrylate, 2-hydroxyethyl(meth)acrylate, 2-hydroxypropyl(meth)acrylate, methoxypolyethyleneglycol (meth)acrylate, and polyethyleneglycol (meth)acrylate; and amino group-containing unsaturated monomers and quaternary salts thereof such as (N,N)-dimethylaminoethyl (meth)acrylate and (N,N)-dimethylaminopropyl (meth)acrylamide. As such, acrylic acid or salts thereof are more preferably used. The use of acrylic acid or salts thereof as the monomer is advantageous because a superabsorbent resin having improved absorbability may be obtained.

In the method of preparing the antibacterial superabsorbent resin according to the present invention, the concentration of the water-soluble ethylenic unsaturated monomer of the monomer composition may be appropriately determined in consideration of the polymerization time and the reaction conditions, and is preferably set to 40 to 55 wt%. If the concentration of the water-soluble ethylenic unsaturated monomer is less than 40 wt%, economic benefits are negated. On the other hand, if the concentration thereof exceeds 55 wt%, the pulverizing efficiency of the hydrogel polymer may decrease.

Whether the hydrogel polymer is prepared from the monomer composition using thermal polymerization or photopolymerization is not limited, so long as the method is typically useful. Specifically, the polymerization method may include thermal polymerization and photopolymerization, depending on the source of energy used for polymerization. Typically, thermal polymerization may be conducted using a reactor having a stirring shaft, such as a kneader, and photopolymerization may be carried out using a reactor having a movable conveyor belt. However, the above polymerization methods are merely illustrative, and the present invention is not limited thereto.

For example, hot air may be fed into a reactor with a stirring shaft, such as a kneader, or the reactor may be heated, so that thermal polymerization is carried out, yielding a hydrogel polymer, which may then be discharged at a size ranging from ones of mm to ones of cm through the outlet of the reactor, depending on the shape of the stirring shaft of the reactor. Specifically, the size of the hydrogel polymer may vary depending on the concentration of the supplied monomer composition and the supply rate thereof, and typically a hydrogel polymer having a particle size of 2 to 50 mm may be obtained.

Also, when photopolymerization is carried out using a reactor having a movable conveyor belt, a hydrogel polymer in sheet form having the same width as the belt may result. As such, the thickness of the polymer sheet may vary depending on the concentration of the supplied monomer composition and the supply rate thereof, but the monomer composition is preferably supplied so as to form a polymer sheet having a thickness of 0.5 to 5 cm. In the case where the monomer composition is supplied to an extent that a very thin polymer sheet is formed, production efficiency may decrease, which is undesirable. If the thickness of the polymer sheet exceeds 5 cm, polymerization may not be uniformly carried out throughout the sheet, which is too thick.

The hydrogel polymer thus obtained typically has a moisture content of 30 to 60 wt%. As used herein, the term "moisture content" refers to an amount of moisture based on the total weight of the hydrogel polymer, that is, a value obtained by subtracting the weight of the dried polymer from the weight of the hydrogel polymer. (Specifically, it is defined as a value calculated by measuring the weight lost from the polymer due to the evaporation of moisture while drying the polymer at a high temperature via IR heating. As such, the drying is performed in such a manner that the temperature is increased from room temperature to 180°C and then maintained at 180°C, and the total drying time is set to 20 min, including 5 min necessary for increasing the temperature.)

The hydrogel polymer obtained through thermal polymerization or photopolymerization is dried, and the drying temperature is preferably set to 150 to 250°C. As used herein, the term "drying temperature" refers to the temperature of a heat medium supplied for the drying process or the temperature of a drying reactor containing a heat medium and a polymer in the drying process.

If the drying temperature is lower than 150°C, the drying time may become excessively long, and the properties of the final superabsorbent resin may thus be deteriorated. On the other hand, if the drying temperature is higher than 250°C, only the surface of the polymer may be excessively dried, whereby a fine powder may be generated in the subsequent pulverizing process and the properties of the final superabsorbent resin may be deteriorated. The drying is preferably performed at a temperature of 150 to 250°C, and more preferably 160 to 200°C.

The drying time is not limited, but may be set to the range of 20 to 90 min, taking processing efficiency into account.

Also, the drying process is not limited, so long as it is typically used to dry the hydrogel polymer. Specific examples thereof may include hot air supply, IR irradiation, microwave irradiation, and UV irradiation. After the drying process, the polymer may have a moisture content of 0.1 to 10 wt%.

Meanwhile, the method of preparing the antibacterial superabsorbent resin according to the present invention may further include a simple pulverizing process before the drying process, as necessary, in order to increase the drying efficiency. The simple pulverizing process is conducted before the drying process so that the particle size of the hydrogel polymer falls in the range of 1 to 15 mm. Pulverizing the particle size of the polymer to less than 1 mm is technically difficult attributable to the high moisture content of the hydrogel polymer, and the pulverized particles may agglomerate. On the other hand, if the polymer is pulverized to a particle size exceeding 15 mm, the effect of increasing the efficiency of the drying process following the pulverizing process may become insignificant.

In the simple pulverizing process that precedes the drying process, any pulverizer may be used without limitation. A specific example thereof may include, but is not limited to, any one selected from the group consisting of a vertical pulverizer, a turbo cutter, a turbo grinder, a rotary cutter mill, a cutter mill, a disc mill, a shred crusher, a crusher, a chopper, and a disc cutter.

When the pulverizing process is performed to increase the drying efficiency before the drying process in this way, the polymer, which has high moisture content, may stick to the surface of the pulverizer. Hence, in order to increase the pulverizing efficiency of the hydrogel polymer before the drying process, an additive able to prevent stickiness may be further used upon pulverizing.

The specific kind of additive that may be found useful is not limited. Examples thereof may include, but are not limited to, a fine-powder agglomeration inhibitor, such as steam, water, a surfactant, and inorganic powder such as clay or silica; a thermal polymerization initiator, such as a persulfate-based initiator, an azo-based initiator, hydrogen peroxide, and ascorbic acid; and a crosslinking agent, such as an epoxy-based crosslinking agent, a diol-based crosslinking agent, a bifunctional or trifunctional or higher polyfunctional acrylate, and a monofunctional compound having a hydroxyl group.

After the drying process, the dried hydrogel polymer is pulverized. The polymer resulting from such a pulverizing process has a particle size of 150 to 850 $\mu$m, and is referred to as a base polymer. The base polymer may be prepared into a superabsorbent resin through drying, pulverizing, classifying, and surface-crosslinking.

The pulverizer used to pulverize the hydrogel polymer to this particle size may include, but is not limited to, a pin mill, a hammer mill, a screw mill, a roll mill, a disc mill, or a jog mill.

The method of preparing the antibacterial superabsorbent resin according to the present invention may further comprise surface-crosslinking the superabsorbent resin using a surface-crosslinking agent.

The surface-crosslinking agent may include at least one selected from the group consisting of water, an alcohol compound, an epoxy compound, a polyamine compound, a haloepoxy compound, a haloepoxy compound condensed product, an oxazoline compound, a mono-, di- or poly-oxazolidinone compound, a cyclic urea compound, a multivalent metal salt, particles having i) a BET specific surface area of 300 to 1500 $m^2$/g and ii) a porosity of 50% or more, an organic carboxylic acid compound, and an alkylene carbonate compound. Preferably useful is at least one selected from the group consisting of water, methanol, particles having i) a BET specific surface area of 300 to 1500 $m^2$/g and ii) a porosity of 50% or more, and oxalic acid.

The particles are not limited so long as they have any one selected from among properties including i) a BET specific surface area of 300 to 1500 $m^2$/g, ii) a porosity of 50% or more, iii) a particle size ranging from 2 nm to 50 $\mu$m, and iv) superhydrophobicity with a water contact angle of 125° or more.

Specifically, the particles preferably include at least one selected from the group consisting of silica ($SiO_2$), alumina, carbon, and titania ($TiO_2$). Most preferably useful is silica ($SiO_2$).

Specifically, the alcohol compound may include at least one selected from the group consisting of methanol, ethanol, propanol, mono-, di-, tri-, tetra- or poly-ethylene glycol, monopropylene glycol, 1,3-propanediol, dipropylene glycol, 2,3,4-trimethyl-1,3-pentanediol, polypropylene glycol, glycerol, polyglycerol, 2-butene-1,4-diol, 1,4-butanediol, 1,3-butanediol, 1,5-pentanediol, 1,6-hexanediol, and 1,2-cyclohexanedimethanol.

Examples of the epoxy compound may include ethylene glycol diglycidyl ether and glycidol, and the polyamine compound may include at least one selected from the group consisting of ethylene diamine, diethylene triamine, triethylene tetramine, tetraethylene pentamine, pentaethylene hexamine, polyethyleneimine, and polyamide polyamine.

Examples of the haloepoxy compound may include epichlorohydrin, epibromohydrin, and $\alpha$-methyl epichlorohydrin. The mono-, di- or poly-oxazolidinone compound may be exemplified by 2-oxazolidinone. The alkylene carbonate compound may include ethylene carbonate. These compounds may be used alone or in combination. To increase the efficiency of the surface-crosslinking process, the surface-crosslinking agent preferably includes, but is not limited to, at least one alcohol compound.

In an embodiment of the present invention, the amount of the surface-crosslinking agent added to treat the surface of the polymer particles may be appropriately determined depending on the kind of surface-crosslinking agent or the reaction conditions, and is set to 0.001 to 5 parts by weight, preferably 0.01 to 3 parts by weight, and more preferably 0.05 to 2 parts by weight, based on 100 parts by weight of the superabsorbent resin.

If the amount of the surface-crosslinking agent is too small, the surface-crosslinking reaction does not readily occur. On the other hand, if the amount thereof exceeds 5 parts by weight based on 100 parts by weight of the polymer, the properties of the superabsorbent resin may deteriorate due to excessive surface-crosslinking reactions.

Here, the method whereby the surface-crosslinking agent is added to the polymer is not limited. Specifically, the surface-crosslinking agent and the polymer powder may be placed in a reaction bath and mixed, the surface-crosslinking agent may be sprayed onto the polymer powder, or the polymer and the crosslinking agent may be continuously supplied and mixed using a reaction bath, such as a mixer that operates continuously.

The temperature of the polymer itself may be 20 to 90°C when the surface-crosslinking agent is added, so that the temperature is increased to the reaction temperature within 1 to 60 min to perform a surface-crosslinking reaction in the presence of the surface-crosslinking agent. To realize the above temperature of the polymer itself, processes after the drying process, which is carried out at a relatively high temperature, are continuously performed, and the processing time may be shortened. Alternatively, the polymer may be heated separately when it is difficult to shorten the processing time.

In the method of preparing the antibacterial superabsorbent resin according to the present invention, the surface-crosslinking agent added to the polymer may be heated, so that the temperature is increased to the reaction temperature within 1 to 60 min to perform a surface-crosslinking reaction in the presence of the surface-crosslinking agent.

In another embodiment of the present invention, when the surface-crosslinking agent is added, the surface temperature of the polymer preferably falls in the range of 60 to 90°C, and the temperature of the surface-crosslinking agent preferably falls in the range of 5 to 40°C, but the present invention is not limited thereto.

More specifically, in the method of preparing the antibacterial superabsorbent resin according to the present invention, when the surface-crosslinking reaction is carried out after increasing the temperature to the reaction temperature within 1 to 60 min so as to prepare for a surface-crosslinking reaction, the efficiency of the surface-crosslinking process may be increased. Ultimately, the residual monomer content of the final superabsorbent resin may be minimized, and a superabsorbent resin having superior properties may be attained. As such, the temperature of the added surface-crosslinking agent is adjusted within the range from 5 to 60°C, and preferably 10 to 40°C. If the temperature of the surface-crosslinking agent is lower than 5°C, the heating rate reduction effect may become insignificant in terms of realizing the surface-crosslinking reaction via heating of the surface-crosslinking agent. On the other hand, if the temperature of the surface-crosslinking agent is higher than 60°C, the surface-crosslinking agent may not be uniformly dispersed in the polymer. As used herein, the surface-crosslinking reaction temperature may be defined as the combined temperature of the polymer and the surface-crosslinking agent that is added for the crosslinking reaction.

The heating member for the surface-crosslinking reaction is not limited. Specifically, a heat medium may be supplied, or direct heating may be conducted using electricity, but the present invention is not limited thereto. Specific examples of the heat source may include steam, electricity, UV light, and IR light. Additionally, a heated thermal fluid may be used.

In the method of preparing the antibacterial superabsorbent resin according to the present invention, after heating for the surface-crosslinking reaction, the surface-crosslinking reaction is carried out for 1 to 120 min, preferably 5 to 40 min, and more preferably 10 to 20 min. If the surface-crosslinking reaction time is less than 1 min, the crosslinking reaction may not sufficiently occur. On the other hand, if the crosslinking reaction time exceeds 60 min, the properties of the superabsorbent resin may deteriorate due to the excessive surface-crosslinking reaction, and attrition of the polymer may occur due to long-term residence in the reactor.

Also, the superabsorbent resin produced by reacting the hydrogel polymer with the surface-crosslinking agent may be further pulverized. The particle size of the superabsorbent resin thus pulverized ranges from 150 to 850 $\mu$m. Specific examples of pulverizers used to obtain such a particle size may include, but are not limited to, a pin mill, a hammer mill, a screw mill, a roll mill, a disc mill, and a jog mill.

In addition, provided is a composition for preparing an antibacterial superabsorbent resin, comprising a fine-powder regranulate and a copper-based antibacterial agent, wherein the fine-powder regranulate is a mixture which includes a fine powder having a particle size of less than 150 $\mu$m, obtained by subjecting a hydrogel polymer to drying, pulverizing and classifying.

## Mode for Invention

A better understanding of the present invention may be obtained via the following non-limited examples, which are set forth to illustrate, but are not to be construed as limiting the scope of the present invention. The scope of the present invention is given by the claims, and also contains all modifications within the meaning and range equivalent to the claims. Unless otherwise mentioned, "%" and "part", indicating amounts in the following examples and comparative examples, are given on a mass basis.

Examples

Example 1. Preparation of antibacterial superabsorbent resin

(1) Preparation of base polymer and fine powder

100 g of acrylic acid, 0.3 g of polyethyleneglycol diacrylate as a crosslinking agent, 0.033 g of diphenyl(2,4,6-trimethylbenzoyl)-phosphine oxide as an initiator, 38.9 g of sodium hydroxide (NaOH), and 103.9 g of water were mixed, thus preparing a monomer mixture.

The monomer mixture was then placed on a continuously moving conveyor belt and irradiated with UV light (at 2 mW/cm$^2$) so that UV polymerization was carried out for 2 min, thus obtaining a hydrogel polymer.

The hydrogel polymer thus obtained was cut to a size of 5 x 5 mm, dried in a hot air oven at 170°C for 2 hr, pulverized using a pin mill, and then classified using a standard sieve based on ASTM standards, thereby obtaining a base polymer having a particle size of 150 to 850 $\mu$m and a fine powder having a particle size of less than 150 $\mu$m.

(2) Preparation of fine-powder regranulate

The fine powder prepared in (1) above was placed in a fine-powder granulator, mixed with water and a Cupron powder (available from Cupron) serving as a copper-based antibacterial agent in an amount of 0.25 parts by weight based on the total weight of a mixture of water and fine powder, and then granulated for 1 min by spraying an additive solution comprising 3 wt% of sodium hydroxide (NaOH) and 1500 ppm of sodium persulfate (SPS), resulting in a fine-powder regranulate.

(3) The fine-powder regranulate was passed through a chopper, dried and pulverized, and thus a fine-powder regranulate in powder form was obtained.

(4) Preparation of antibacterial superabsorbent resin

The fine-powder regranulate in powder form was uniformly mixed with a mixture solution comprising 0.3 g of ethylene carbonate, 3.5 g of methanol, 3.0 g of water, 0.22 g of oxalic acid, and 0.01 g of aerogel (available from JIOS), and then allowed to react while drying in a hot air oven. The aerogel had an average particle size of 5 $\mu$m, a BET specific surface area of 720 m$^2$/g, a water contact angle of 144°, and a porosity of 95%. As for the aerogel, the particle size was measured using a HELOS (Helium-Neon Laser Optical System) through laser diffraction in accordance with ISO 13320. The specific surface area was measured using a BET system (Micromeritics 3Flex). The porosity was determined based on the following equation: porosity (%) = (1 - $\rho_t/\rho_s$)*100 (wherein $\rho_t$ is tap density and $\rho_s$ is true density).

Here, the true density was measured using a pycnometer (Accupyc II 1340), and the tap density was measured using a volumeter (Engelsmann Model STAV II).

The water contact angle was measured using a contact angle analyzer (KRUSS DSA100), and was specifically determined in a manner in which a piece of double-sided tape was attached to a flat glass plate, microparticles were applied in a monolayer thereon, and then 5 $\mu$L of ultrapure water was placed in the form of a drop on the monolayer, and the angle between the water drop and the glass plate was measured four times and averaged.

The dried powder was classified using a standard sieve based on ASTM standards, yielding a final antibacterial superabsorbent resin having a particle size of 150 to 850 $\mu$m.

Example 2. Preparation of antibacterial superabsorbent resin

An antibacterial superabsorbent resin was prepared in the same manner as in Example 1, with the exception that the Cupron powder (available from Cupron) was added in an amount of 0.5 parts by weight based on the total weight of the mixture of water and fine powder in (2) of Example 1.

Comparative Example 1. Preparation of superabsorbent resin

An antibacterial superabsorbent resin was prepared in the same manner as in Example 1, with the exception that the Cupron powder (available from Cupron) was added in an amount of 0.1 parts by weight based on the total weight of the mixture of water and fine powder in (2) of Example 1.

Comparative Example 2. Preparation of antibacterial superabsorbent resin

An antibacterial superabsorbent resin was prepared in the same manner as in Example 1, with the exception that the Cupron powder (available from Cupron) was added in an amount of 1.0 part by weight based on the total weight of the mixture of water and fine powder in (2) of Example 1.

Comparative Examples 3 to 8. Superabsorbent resins

Six kinds of superabsorbent resins (A, B, C, D, E, and F, respectively, available from LG Chemicals), having no antibacterial agent, were used in Comparative Examples 3 to 8.

Test Example

Test Example 1. Antibacterial test (Lab scale)

The superabsorbent resins of Examples 1 and 2 and Comparative Examples 1 to 8 were subjected to an antibacterial test.
Artificial urine, manufactured to evaluate antibacterial effects, was mixed with a nutrient solution to give a culture medium, on which *E. Coli* (ATCC 8739) was then cultured, thus obtaining a bacterial solution. 40 g of the superabsorbent resin (SAP) of each of Examples 1 and 2 and Comparative Examples 1 to 8 and the bacterial solution were stirred, after which the bacterial solution attached to the superabsorbent resin was diluted, inoculated to an agar medium, and cultured. The concentration of cultured bacteria was calculated and Colony Forming Units (CFUs) were analyzed.
The results are shown in Table 1 below.

[Table 1]

| | 0 hr *E. Coli* concentration (CFU/ml) | 24 hr *E. Coli* concentration (CFU/ml) | Reduction (%) |
|---|---|---|---|
| Ex.1 | $2.65 \times 10^6$ | $1.56 \times 10^5$ | 99.99 |
| Ex.2 | $2.65 \times 10^6$ | $3.33 \times 10^2$ | 99.99 |
| C.Ex.1 | $2.65 \times 10^6$ | $3.33 \times 10^2$ | 94.13 |
| C.Ex.2 | $2.65 \times 10^6$ | $3.33 \times 10^2$ | 99.99 |
| C.Ex.3 (A) | $7.07 \times 10^6$ | $3.93 \times 10^6$ | 39.20 |
| C.Ex.4 (B) | $7.07 \times 10^6$ | $1.06 \times 10^7$ | -63.32 |
| C.Ex.5 (C) | $7.07 \times 10^6$ | $1.06 \times 10^7$ | -65.38 |
| C.Ex.6 (D) | $7.07 \times 10^6$ | $6.93 \times 10^6$ | -7.16 |
| C.Ex.7 (E) | $7.07 \times 10^6$ | $8.80 \times 10^6$ | -36.01 |
| C.Ex.8 (F) | $7.07 \times 10^6$ | $9.03 \times 10^6$ | -39.62 |

As is apparent from Table 1, the superabsorbent resins (Examples 1 and 2 and Comparative Example 2) containing 0.25 parts by weight or more of Cupron exhibited high antibacterial effects.

Test Example 2. Antibacterial test (Pilot scale)

An antibacterial test was performed in the same manner as in Test Example 1, with the exception that 2 kg of the superabsorbent resin (SAP) of each of Example 2 and Comparative Example 2 was used.
The results are shown in Table 2 below.

[Table 2]

| | 0 hr *E. Coli* concentration (CFU/ml) | 24 hr *E. Coli* concentration (CFU/ml) | Reduction (%) |
|---|---|---|---|
| Ex.2 | $4.53 \times 10^5$ | $1.30 \times 10^4$ | 97.13 |

(continued)

|  | 0 hr *E. Coli* concentration | 24 hr *E. Coli* concentration | Reduction |
|---|---|---|---|
|  | (CFU/ml) | (CFU/ml) | (%) |
| C.Ex.2 | $3.57 \times 10^6$ | $1.67 \times 10^4$ | 99.53 |

On a pilot scale, the antibacterial effects were lower than on the lab scale, but the sample of Example 2, containing 0.5 parts by weight or more of Cupron, exhibited high antibacterial effects.

Test Example 3. Centrifugal Retention Capacity (CRC)

The superabsorbent resins of Examples 1 and 2 and Comparative Examples 1 and 2 were measured for CRC. CRC was measured using the European Disposables and Nonwovens Association (EDANA) method WSP 241.3 (10) (IST 241.2(02)). Specifically, W (g) (about 0.2 g) of the superabsorbent resin of each of Examples 1 and 2 and Comparative Examples 1 and 2 was uniformly placed in a bag made of nonwoven fabric, sealed, and immersed in a 0.9 mass% saline solution at room temperature. After 30 min, the bag was centrifuged at 250G for 3 min to remove water, and the mass W2 (g) of the bag was measured. Also, the same procedures were performed without the use of the polymer, after which the mass W1 (g) was measured. The mass values thus obtained were substituted into the following Equation 1, thus determining CRC (g/g).

$$[\text{Equation 1}]$$
$$\text{CRC (g/g)} = \{(W2\ (g) - W1\ (g))/W\ (g)\} - 1$$

The test results are shown in Table 3 below.

Test Example 4. Absorption Under Pressure (AUP)

The superabsorbent resins of Examples 1 and 2 and Comparative Examples 1 and 2 were measured for AUP. AUP was measured using the EDANA method WSP 242.3 (11) (IST 242.2(02)).
Specifically, a 400 mesh iron net made of stainless steel was mounted to the bottom of a plastic cylinder having an inner diameter of 60 mm. 0.90 g of the superabsorbent resin of each of Examples 1 and 2 and Comparative Examples 1 and 2 was uniformly sprayed onto the net at room temperature under a humidity of 50%, after which a load of 4.83 kPa (0.7 psi) was uniformly applied thereto using a piston having an outer diameter slightly smaller than 60 mm without leaving any gap with the inner wall of the cylinder but without disturbing the up-down movement. The weight Wa (g) of the above device was measured.
A glass filter having a diameter of 90 mm and a thickness of 5 mm was placed in a Petri dish having a diameter of 150 mm, and a saline solution composed of 0.90 wt% sodium chloride was added to be flush with the upper surface of the glass filter. Then, a filter paper having a diameter of 90 mm was placed thereon, and the above measurement device was placed on the filter paper and was allowed to absorb a liquid for 1 hr under a predetermined load. After 1 hr, the measurement device was removed, and the weight Wb (g) was measured.
The Wa and Wb values were substituted into the following Equation 2 to determine AUP.

$$[\text{Equation 2}]$$
$$\text{AUP (g/g)} = [Wb(g) - Wa(g)]/\text{mass (g) of absorbent resin}$$

Test results are shown in Table 3 below.

Test Example 5. Absorption Speed

The superabsorbent resins of Examples 1 and 2 and Comparative Examples 1 and 2 were measured for absorption speed. 50 mL of saline was placed in a 100 mL beaker together with a magnetic bar. The stirring rate was set to 600 rpm using a stirrer. The time was measured at the time at which 2.0 g of the superabsorbent resin was added to the saline, which was stirred. The measurement of the time was terminated when the vortex in the beaker disappeared.
Test results are shown in Table 3 below.

Test Example 6. Permeability

The superabsorbent resins of Examples 1 and 2 and Comparative Examples 1 and 2 were measured for permeability.

In order to prevent the generation of bubbles between a cock and a glass filter in the lower portion of a chromatography column, about 10 mL of water was added in the opposite direction into the column, and the column was washed two or three times with saline and then filled with at least 40 mL of 0.9% saline. A piston was placed in the chromatography column, the lower valve was opened, and the time (B: sec) required for the liquid surface to move from 40 mL to 20 mL was recorded, thus completing blank testing. 0.2 g of a sample of the prepared superabsorbent resin, having a particle size ranging from 300 to 600 $\mu$m, was placed in the column, and then saline was added such that the total amount of saline that resulted was 50 mL, after which the sample was allowed to stand for 30 min so that the superabsorbent resin was sufficiently swollen. Thereafter, a weighted piston (0.3 psi) was placed in the chromatography column and then allowed to stand for 1 min. The cock at the bottom of the chromatography column was opened, and the time (T1: sec) required for the liquid surface to move from 40 mL to 20 mL was recorded. The permeability was determined based on the following equation.

$$\text{Permeability} = T1 - B$$

Test results are shown in Table 3 below.

[Table 3]

|  | CRC | AUP | Absorption speed | Permeability |
|---|---|---|---|---|
| Ex.1 | 25.5 | 20.9 | 35 | 156 |
| Ex.2 | 25.8 | 19.7 | 36 | 210 |
| C.Ex.1 | 26.4 | 18.2 | 39 | 390 |
| C.Ex.2 | 27.6 | 16.8 | 48 | 908 |

As is apparent from the results of measurement of the properties as above, when the amount of Cupron that was added was increased, AUP and permeability decreased. CRC and absorption speed did not exhibit a clear trend.

Based on the above test results, in order to realize the effects of the present invention for minimizing the deterioration of properties of the superabsorbent resin and imparting antibacterial activity, as in Examples 1 and 2, the superabsorbent resin was added with the copper-based antibacterial agent in an amount of preferably 0.2 to 0.9 parts by weight, and more preferably 0.5 to 0.8 parts by weight, based on 100 parts by weight of the superabsorbent resin.

Examples 1 and 2 were measured for absorption speed. 50 mL of saline was placed in a 100 mL beaker together with a magnetic bar. The stirring rate was set to 600 rpm using a stirrer. The time was measured at the time at which 2.0 g of the superabsorbent resin was added to the saline, which was stirred. The measurement of the time was terminated when the vortex in the beaker disappeared.

Test results are shown in Table 3 below.

Test Example 6. Permeability

The superabsorbent resins of Examples 1 and 2 and Comparative Examples 1 and 2 were measured for permeability.

In order to prevent the generation of bubbles between a cock and a glass filter in the lower portion of a chromatography column, about 10 mL of water was added in the opposite direction into the column, and the column was washed two or three times with saline and then filled with at least 40 mL of 0.9% saline. A piston was placed in the chromatography column, the lower valve was opened, and the time (B: sec) required for the liquid surface to move from 40 mL to 20 mL was recorded, thus completing blank testing. 0.2 g of a sample of the prepared superabsorbent resin, having a particle size ranging from 300 to 600 $\mu$m, was placed in the column, and then saline was added such that the total amount of saline that resulted was 50 mL, after which the sample was allowed to stand for 30 min so that the superabsorbent resin was sufficiently swollen. Thereafter, a weighted piston (0.3 psi) was placed in the chromatography column and then allowed to stand for 1 min. The cock at the bottom of the chromatography column was opened, and the time (T1: sec) required for the liquid surface to move from 40 mL to 20 mL was recorded. The permeability was determined based on the following equation.

$$\text{Permeability} = T1 - B$$

Test results are shown in Table 3 below.

[Table 3]

|  | CRC | AUP | Absorption speed | Permeability |
|---|---|---|---|---|
| Ex.1 | 25.5 | 20.9 | 35 | 156 |
| Ex.2 | 25.8 | 19.7 | 36 | 210 |
| C.Ex.1 | 26.4 | 18.2 | 39 | 390 |
| C.Ex.2 | 27.6 | 16.8 | 48 | 908 |

As is apparent from the results of measurement of the properties as above, when the amount of Cupron that was added was increased, AUP and permeability decreased. CRC and absorption speed did not exhibit a clear trend.
Based on the above test results, in order to realize the effects of the present invention for minimizing the deterioration of properties of the superabsorbent resin and imparting antibacterial activity, as in Examples 1 and 2, the superabsorbent resin was added with the copper-based antibacterial agent in an amount of preferably 0.2 to 0.9 parts by weight, and more preferably 0.5 to 0.8 parts by weight, based on 100 parts by weight of the superabsorbent resin.

**Claims**

1. A method of preparing an antibacterial superabsorbent resin, comprising:

    1) drying and pulverizing a hydrogel polymer, and then classifying into a fine powder having a particle size of less than 150 μm and a base polymer having a particle size of 150 to 850 μm;
    2) mixing the fine powder, water and a copper-based antibacterial agent, thus preparing a fine-powder regranulate;
    3) passing the fine-powder regranulate through a chopper and then performing drying and pulverizing; and
    4) classifying the pulverized fine-powder regranulate into a superabsorbent resin having a particle size of 150 to 850 μm, thus obtaining the superabsorbent resin.

2. The method of claim 1, wherein the copper-based antibacterial agent includes $Cu_2O$ (cuprous oxide).

3. The method of claim 1, wherein the copper-based antibacterial agent is added in an amount of 0.2 to 0.9 parts by weight based on 100 parts by weight of the superabsorbent resin.

4. The method of claim 1, wherein the copper-based antibacterial agent exhibits antibacterial activity against gram-negative bacteria, such as *Escherichia coli.*

5. The method of claim 1, wherein the mixing in 2) further comprises mixing at least one additive selected from the group consisting of sodium hydroxide (NaOH) and sodium persulfate (SPS).

6. The method of claim 1, further comprising surface-crosslinking the superabsorbent resin with a surface-crosslinking agent.

7. The method of claim 6, wherein the surface-crosslinking agent includes at least one selected from the group consisting of water; an alcohol compound; an epoxy compound; a polyamine compound; a haloepoxy compound; a haloepoxy compound condensed product; an oxazoline compound; a mono-, di- or poly-oxazolidinone compound; a cyclic urea compound; a multivalent metal salt; particles having i) a BET specific surface area of 300 to 1500 $m^2$/g and ii) a porosity of 50% or more; an organic carboxylic acid compound; and an alkylene carbonate compound.

8. The method of claim 6, wherein the surface-crosslinking agent includes at least one selected from the group consisting of ethylene carbonate, water, methanol, particles having i) a BET specific surface area of 300 to 1500 $m^2$/g and ii) a porosity of 50% or more, and oxalic acid.

**Patentansprüche**

1. Verfahren zum Herstellen eines antibakteriellen Superabsorberharzes, umfassend:

   1) Trocknen und Pulverisieren eines Hydrogelpolymers, und dann Klassieren in ein feines Pulver mit einer Teilchengröße von weniger als 150 $\mu$m und ein Basispolymer mit einer Teilchengröße von 150 bis 850 $\mu$m;
   2) Mischen des feinen Pulvers, Wasser und eines antibakteriellen Mittels auf Kupferbasis, somit herstellend ein Feinpulverregranulat;
   3) Führen des Feinpulverregranulats durch ein Zerkleinerungsgerät und dann Durchführen von Trocknen und Pulversieren; und
   4) Klassieren des pulverisierten Feinpulverregranulats in ein Superabsorberharz mit einer Teilchengröße von 150 bis 850 $\mu$m, somit erhaltend das Superabsorberharz.

2. Verfahren nach Anspruch 1, wobei das antibakterielle Mittel auf Kupferbasis $Cu_2O$ (Kupfer(I)oxid) einschließt.

3. Verfahren nach Anspruch 1, wobei das antibakterielle Mittel auf Kupferbasis in einer Menge von 0,2 bis 0,9 Gewichtsteilen, basierend auf 100 Gewichtsteilen des Superabsorberharzes, zugegeben wird.

4. Verfahren nach Anspruch 1, wobei das antibakterielle Mittel auf Kupferbasis antibakterielle Aktivität gegen Gram-negative Bakterien, wie *Escherichia coli,* zeigt.

5. Verfahren nach Anspruch 1, wobei das Mischen in 2) ferner ein Mischen wenigstens eines Additivs, ausgewählt aus der Gruppe bestehend aus Natriumhydroxid (NaOH) und Natriumpersulfat (SPS), umfasst.

6. Verfahren nach Anspruch 1, weiter umfassend ein Oberflächenvernetzen des Superabsorberharzes mit einem Oberflächenvernetzungsmittel.

7. Verfahren nach Anspruch 6, wobei das Oberflächenvernetzungsmittel wenigstens eines einschließt, ausgewählt aus der Gruppe bestehend aus Wasser; einer Alkoholverbindung; einer Epoxyverbindung; einer Polyaminverbindung; einer Halogenepoxyverbindung; einem kondensierten Produkt einer Halogenepoxyverbindung; einer Oxazolinverbindung; einer Mono-, Di- oder Poly-oxazolidinonverbindung; einer zyklischen Harnstoffverbindung; einem mehrwertigen Metallsalz; Teilchen mit i) einer spezifischen BET-Oberfläche von 300 bis 1500 m$^2$/g und ii) einer Porosität von 50% oder mehr; einer organischen Carbonsäureverbindung; und einer Alkylencarbonatverbindung.

8. Verfahren nach Anspruch 6, wobei das Oberflächenvernetzungsmittel wenigstens eines einschließt, ausgewählt aus der Gruppe bestehend aus Ethylencarbonat, Wasser, Methanol, Teilchen mit i) einer spezifischen BET-Oberfläche von 300 bis 1500 m$^2$/g und ii) einer Porosität von 50% oder mehr, und Oxalsäure.

**Revendications**

1. Procédé de préparation d'une résine superabsorbante antibactérienne, consistant à :

   1) sécher et pulvériser un polymère hydrogel, puis classer en une poudre fine ayant une dimension de particule inférieure à 150 $\mu$m et un polymère de base ayant une dimension de particule comprise entre 150 et 850 $\mu$m;
   2) mélanger la poudre fine, de l'eau et un agent antibactérien à base de cuivre, et ainsi préparer un regranulat en poudre fine;
   3) passer le regranulat en poudre fine dans un hacheur, puis effectuer le séchage et la pulvérisation; et
   4) classer le regranulat en poudre fine pulvérisé en une résine superabsorbante ayant une dimension de particule comprise entre 150 et 850 $\mu$m, et ainsi obtenir la résine superabsorbante.

2. Procédé selon la revendication 1, dans lequel l'agent antibactérien à base de cuivre inclut $Cu_2O$ (oxyde cuivreux).

3. Procédé selon la revendication 1, dans lequel l'agent antibactérien à base de cuivre est ajouté dans une quantité comprise entre 0,2 et 0,9 partie en poids sur la base de 100 parties en poids de la résine superabsorbante.

4. Procédé selon la revendication 1, dans lequel l'agent antibactérien à base de cuivre présente une activité antibactérienne contre les bactéries à gram négatif, tel le *Escherichia coli.*

**5.** Procédé selon la revendication 1, dans lequel le mélangeage en 2) consiste en outre à mélanger un ou plusieurs additifs sélectionnés dans le groupe constitué de hydroxyde de sodium (NaOH) et persulfate de sodium (SPS).

**6.** Procédé selon la revendication 1, consistant en outre à réticuler de surface la résine superabsorbante avec un agent de réticulation de surface.

**7.** Procédé selon la revendication 6, dans lequel l'agent de réticulation de surface inclut un ou plusieurs agents sélectionnés dans le groupe constitué d'eau; un composé d'alcool; un composé d'époxy ; un composé de polyamine ; un composé d'haloépoxy; un produit de condensation d'un composé d'haloépoxy ; un composé d'oxazoline ; un composé de mono-, di- ou polyoxazolidinone; un composé d'urée cyclique ; un sel de métal polyvalent; des particules ayant i) une surface spécifique BET comprise entre 300 et 1500 m$^2$/g et ii) une porosité égale ou supérieure à 50 %; un composé d'acide carboxylique organique; et un composé de carbonate d'alkylène.

**8.** Procédé selon la revendication 6, dans lequel l'agent de réticulation de surface inclut un ou plusieurs agents sélectionnés dans le groupe constitué de carbonate d'éthylène, d'eau, de méthanol, de particules ayant i) une surface spécifique BET comprise entre 300 et 1500 m$^2$/g et ii) une porosité égale ou supérieure à 50 %, et d'acide oxalique.

【FIG. 1】

FIG. 1

**REFERENCES CITED IN THE DESCRIPTION**

**Patent documents cited in the description**

- EP 1616911 A1
- JP H09143850 A
- US 2014221948 A1